# EUROPEAN PATENT SPECIFICATION

(11) **EP 2 593 570 B1**
(45) Date of publication and mention of the grant of the patent: **01.03.2017**
(21) Application number: 11754750.5
(22) Date of filing: 11.07.2011
(51) Int. Cl.: C12N 1/20, A23C 11/10, A23L 11/00, A23L 33/105, A23L 33/135, A61K 8/64, A61K 8/97, A61K 31/352, A61K 31/353, A61K 35/747, A61K 36/48, A61K 38/16, A61P 1/00, A61P 17/14, A61Q 7/00, A61Q 19/00, C12R 1/25, C12R 1/225

(54) **FERMENTED SOY-BASED MIXTURE COMPRISING ISOFLAVONES-AGLYCONES, EQUOL AND LUNASIL, PROCESS FOR THE PREPARATION AND USES THEREOF IN FOOD, MEDICAL AND COSMETIC FIELDS**
FERMENTIERTE SOJAMISCHUNG MIT ISOFLAVON-AGLYCONEN, EQUOL UND LUNASIL, VERFAHREN ZU IHRER HERSTELLUNG UND IHRE VERWENDUNG IM NAHRUNGSMITTELTECHNISCHEN, MEDIZINISCHEN UND KOSMETIKBEREICH
MÉLANGE À BASE DE SOJA FERMENTÉ COMPRENANT DES ISOFLAVONES-AGLYCONES, DE L'ÉQUOL ET DU LUNASIL, PROCÉDÉ POUR LA PRÉPARATION ET UTILISATIONS DE CELUI-CI DANS LES DOMAINES ALIMENTAIRE, MÉDICAL ET COSMÉTIQUE

(30) Priority: 12.07.2010 IT RM20100378
(43) Date of publication of application: 22.05.2013
(73) Proprietor: Giuliani S.p.A., 20129 Milano (IT)
(72) Inventor: GIULIANI, Giammaria, I-20129 Milano (IT); BENEDUSI, Anna, I-20129 Milano (IT); GOBBETTI, Marco, I-20129 Milano (IT); DI CAGNO, Raffaella, I-20129 Milano (IT); BARONI, Sergio, I-20129 Milano (IT); RIZZELLO, Carlo Giuseppe, I-70125 Bari (IT)
(74) Representative: Gitto, Serena
(86) International application number: PCT/IT2011/000240
(87) International publication number: WO 2012/007978

(56) References cited:
- EP-A1- 0 855 140
- EP-A1- 1 120 108
- WO-A1-98/42200
- WO-A1-99/36050
- WO-A1-2008/003782
- FR-A1- 2 831 395
- US-A- 5 026 553
- US-A1- 2006 251 750
- PYO Y H ET AL: "Enrichment of bioactive isoflavones in soymilk fermented with beta-glucosidase-producing lactic acid bacteria", FOOD RESEARCH INTERNATIONAL, vol. 38, no. 5, 1 June 2005 (2005-06-01), pages 551-559, XP004804764, ISSN: 0963-9969, DOI: DOI:10.1016/J.FOODRES.2004.11.008
- REKHA C R ET AL: "Bioconversion of isoflavone glycosides to aglycones, mineral bioavailability and vitamin B complex in fermented soymilk by probiotic bacteria and yeast", JOURNAL OF APPLIED MICROBIOLOGY, vol. 109, no. 4, October 2010 (2010-10), pages 1198-1208, XP002627003, ISSN: 1364-5072
- MARAZZA J A ET AL: "Aglycone production by Lactobacillus rhamnosus CRL981 during soymilk fermentation", FOOD MICROBIOLOGY, vol. 26, no. 3, 1 May 2009 (2009-05-01), pages 333-339, XP026006051, ISSN: 0740-0020, DOI: DOI:10.1016/J.FM.2008.11.004 [retrieved on 2008-12-16]
- CHIEN H-L ET AL: "Transformation of isoflavone phytoestrogens during the fermentation of soymilk with lactic acid bacteria and bifidobacteria", FOOD MICROBIOLOGY, vol. 23, no. 8, 1 December 2006 (2006-12-01), pages 772-778, XP024904367, ISSN: 0740-0020, DOI: DOI:10.1016/J.FM.2006.01.002 [retrieved on 2006-12-01]
- MITAL B K ET AL: "UTILIZATION OF OLIGOSACCHARIDES BY LACTIC ACID BACTERIA DURING FERMENTATION OF SOY MILK", JOURNAL OF FOOD SCIENCE, vol. 40, no. 1, 1 January 1975 (1975-01-01), pages 114-118, XP008005527, ISSN: 0022-1147, DOI: DOI:10.1111/J.1365-2621.1975.TB03749.X
- WEI ET AL: "Using of Lactobacillus and Bifidobacterium to product the isoflavone aglycones in fermented soymilk", INTERNATIONAL JOURNAL OF FOOD MICROBIOLOGY, vol. 117, no. 1, 10 June 2007 (2007-06-10) , pages 120-124, XP022077953, ISSN: 0168-1605, DOI: DOI:10.1016/J.IJFOODMICRO.2007.02.024 cited in the application
- CHUN JIYEON ET AL: "Conversion of isoflavone glucosides to aglycones in soymilk by fermentation with lactic acid bacteria", JOURNAL OF FOOD SCIENCE, vol. 72, no. 2, March 2007 (2007-03), pages M39-M44, XP002627004, ISSN: 0022-1147 cited in the application
- TSANGALIS DIMITRI ET AL: "Development of an isoflavone aglycone-enriched soymilk using soy germ, soy protein isolate and bifidobacteria", FOOD RESEARCH INTERNATIONAL, vol. 37, no. 4, 2004, pages 301-312, XP002627005, ISSN: 0963-9969 cited in the application
- TSANGALIS DIMITRI ET AL: "Metabolism of oligosaccharides and aldehydes and production of organic acids in soymilk by probiotic bifidobacteria", INTERNATIONAL JOURNAL OF FOOD SCIENCE & TECHNOLOGY, vol. 39, no. 5, May 2004 (2004-05), pages 541-554, XP002627006, ISSN: 0950-5423
- TSANGALIS D ET AL: "Enzymic transformation of isoflavone phytoestrogens in soymilk by beta-glucosidase-producing bifidobacteria.", JOURNAL OF FOOD SCIENCE, vol. 67, no. 8, October 2002 (2002-10), pages 3104-3113, XP002627007, ISSN: 0022-1147 cited in the application
- DE MEJIA ELVIRA GONZALEZ ET AL: "Lunasin concentration in different soybean genotypes, commercial soy protein, and isoflavone products", JOURNAL OF AGRICULTURAL AND FOOD CHEMISTRY, vol. 52, no. 19, 22 September 2004 (2004-09-22), pages 5882-5887, XP002627008, ISSN: 0021-8561
- ROUBOS-VAN DEN HIL P J ET AL: "Fermented soya bean (tempe) extracts reduce adhesion of enterotoxigenic Escherichia coli to intestinal epithelial cells.", JOURNAL OF APPLIED MICROBIOLOGY, vol. 106, no. 3, March 2009 (2009-03), pages 1013-1021, XP002627009, ISSN: 1365-2672
- "The use of fermented soybean extract in combination with polysaccharides to improve skin condition", RESEARCH DISCLOSURE, vol. 510, no. 29, 1 October 2006 (2006-10-01), page 1301, XP007136708, MASON PUBLICATIONS, HAMPSHIRE, GB ISSN: 0374-4353
- MCELWEE K J ET AL: "Dietary soy oil content and soy-derived phytoestrogen genistein increase resistance to alopecia areata onset in C3H/HeJ mice.", EXPERIMENTAL DERMATOLOGY, vol. 12, no. 1, February 2003 (2003-02), pages 30-36, XP002627010, ISSN: 0906-6705
- BARNES STEPHEN: "The Biochemistry, Chemistry and Physiology of the Isoflavones in Soybeans and their Food Products", LYMPHATIC RESEARCH AND BIOLOGY, vol. 8, no. 1, 2010, pages 89-98, XP002627011, ISSN: 1539-6851
- DI CAGNO RAFFAELLA ET AL: "Synthesis of Isoflavone Aglycones and Equol in Soy Milks Fermented by Food-Related Lactic Acid Bacteria and Their Effect on Human Intestinal Caco-2 Cells", JOURNAL OF AGRICULTURAL AND FOOD CHEMISTRY, vol. 58, no. 19, October 2010 (2010-10), pages 10338-10346, XP002627012, ISSN: 0021-8561

## Description

The present invention concerns a fermented soy based mixture comprising isoflavones-aglicones, equol and lunasil, the process for the preparation and uses thereof in food, medical and cosmetic fields. In particular, the present invention concerns a mixture comprising isoflavones-aglicones, such as daidzein, genistein and glycitein, in addition to equol and lunasin, said mixture being based on soy fermented using lactic acid bacteria isolated from food matrices and use of said mixture for protection of skin and adnexa and of human intestinal cells with particular reference to prevention of inflammatory state and protection of barrier functions and hair loss treatment.

Isoflavones are diphenolic compounds naturally occurring in various plants and particularly soy (Tsangalis et al., 2002. Enzymatic transformation of isoflavone phytoestrogens in soymilk by β-glucosidase producing bifidobacteria. Food Res. Int. Sci. 67:3104-3113). Soy derived isoflavones and soy based food products belong to 4 classes of chemical compounds: aglicones, malonyl-, acetyl- and β-glucoside-conjugates (Tsangalis et al., 2002. Enzymatic transformation of isoflavone phytoestrogens in soymilk by β-glucosidase producing bifidobacteria. Food Res. Int. Sci. 67:3104-3113). More than 90% of soy isoflavone total concentration occurs as β-glucoside derivatives. Because of the remarkable hydrophobic character and high molecular mass, β-glucoside derivatives are not absorbed by humans. Therefore in order to be bioavailable and thus metabolised said compounds must be hydrolysed to aglicones. Hydrolysis to aglicones such as daidzeindaidzein, genistein and glycitein occurs during the intestinal passage as result of activity of intestinal and bacterial β-glucosidase. In free form, such aglicones are structurally similar to estrogens and mimic the estradiol function in human body (Setchell and Cassidi, 1999. Dietary isoflavones: biological effects and relevance to human health. J. Nutr. 131:758 - 767). Generally, the consumption of isoflavones-aglicones is associated with the reduction of risk of hormonal pathologies (Kruzer 2000. Hormon effects of soy isoflavones: studies in premenopausal and postmenopausal women. J. Nutr. 130: 660-661), and, with lower incidence of osteoporosis, menopause and mortality from cardiovascular pathologies and cancer (Nagata et al. 1998. Decreased serum total cholesterol concentration is associated with high intake of soy products in Japanase men and women. J. Nutr. 128:209-213).

Equol is an estrogen not steroidal compound belonging to isoflavone class. The main source of equol for humans is soy derivatives, representing most abundant reserve for daidzein and aglicone daidzein, direct precursor thereof (Axelson et al., 1984. Soya - a dietary source of the non-steroidal oestrogen equol in man and animals. J. Endocrinol. 102:49-56). Differently than other isoflavones-aglicones, equol is the only one having a core chiral nucleus resulting from the absence of double bond within heterocyclic ring (Setchell et al., 2002. The clinical importance of the metabolite equol a clue to the effectiveness of soy and its isoflavones. Am. Soc. Nutr. Sci. 125: 3577-3583). Generally, equol is absorbed easily through colon wall and is metabolically inert (Setchell et al., 2002. The clinical importance of the metabolite equol a clue to the effectiveness of soy and its isoflavones. Am. Soc. Nutr. Sci. 125: 3577-3583). Compared to daidzein precursor thereof equol shows an interesting set of properties: higher estrogenic activity (Muthyala et al., 2004. Equol, a natural estrogenic metabolita from soy isoflavones: convenient preparation and resolution of R- and S-equols and their differing binding and biological activity through estrogen receptors alpha and beta. Bioorg. Med. Chem. 12:1559-1567) anti-oxidant activity (Mitchell et al., 1998. Antioxidant efficacy of phytoestrogens in chemical and biological model systems. Arch. Biochem. Biophys. 360:142-148), and antiandrogenic activity (Lund et al., 2004. Equol is a novel anti-androgen that inhibits prostate growth and hormone feedback. Bio. Reprod. 70:1188-1195).

Soy isoflavone metabolism in man is widely documented (Axelson et al., 1984. Soya - a dietary source of the non-steroidal oestrogen equol in man and animals. J. Endocrinol. 102:49-56; Bannwart et al., 1984. Identification of o-desmethylangolensin, a metabolita of daidzein and of matairesinol, one likely plant precursor of the animal lignan enterolactone in human urine. Finn. Chem. Lett. 5:120-125). The ability to metabolise glucoside isoflavones to aglicones and aglicones to equol during intestinal passage is limited only to 30-50% of the western countries population (Frankefeld, et al. 2005. High concordance of daidzein-metabilizing phenotypes in individuals measured 1 to 3 years apart. Brit. J. Nutr. 94:873-876). Two main strategies can be pursued in order to increase the bioavailability of soy derived isoflavones: aglicone and equol enrichment before the consumption or modulation of intestinal microbiota by ingestion of viable and vital bacteria suitable to synthesise in situ such compounds (Tsangalis et al., 2004. Development of an isoflavone aglycone-enriched soymilk using soy germ, soy protein isolate and bifidobacteria. Food Res. Intern. 37:301-312). Various studies (Chun et al., 2007. Conversion of isoflavone glucoside to aglycones in soymilk by fermentation with lactic acid bacteria. J. Food Sci. 72:39-44; Donkor and Shah 2008. Production of β-glucosidase and hydrolysis of isoflavone phytoestrogens by Lactobacillus acidophilus, Bifidobacterium lactis and Lactobacillus casei in soymilk. J. Food Sci. 73:15-20; Pham and Shah 2007. Biotransformation of isoflavone glycosides by Bifidobacterium animalis in soymilk supplemented with skim milk powder. J. Food Sci. 72:316-324; Tsangalis et al., 2002; Tsangalis et al., 2004; Wei et al., 2007. Using Lactobacillus and Bifidobacterium to product the isoflavone algycones in fermented soymilk. Int. J. Food Microbiol.117:120-124) have considered the use of bifidobacteria and lactic acid bacteria for the conversion of glucoside isoflavones to aglicones and/or equol during the fermentation of soy milk. However, some limitations are apparent in these studies: (i) a very limited number of bacterial biotype/species has been considered; (ii) bacteria used for fermentation processes are exclusively originated from human fecal material; (iii) a very limited number of substrates for fermentation, none of which involved the use of organic farming soy flour has been considered; (iv) preparations are based only on isoflavone/aglicones or equol, and in the case of equol production maximum concentration is 0,521 mg/100 ml (Tsangalis et al., 2002. Enzymatic transformation of isoflavone phytoestrogens in soymilk by β-glucosidase producing bifidobacteria. Food Res. Int. Sci. 67:3104-3113); (v) no study tested the biological effectiveness of preparations, in particular for skin protection; and (vi) no study formulated a preparation containing isoflavones-aglicones, equol and lunasin.

Lunasin is a bioactive peptide (43 aminoacid residues, molecular weight about. 5400 Da) identified for the first time in soy (Galvez et al., 2001. Chemopreventive property of a soybean peptide (Lunasin) that binds to deacetylated histones and inhibits acetylation. Cancer Res. 61:7473-7478) and successively found also in barley (Jeong et al. 2002. Barley lunasin suppresses ras-induced colony formation and inhibits core histone acetylation in mammalian cells. J. Agric. Food Chem. 50:5903-5908), wheat (Jeong et al. 2007. The cancer preventive peptide lunasin from wheat inhibits core histone acetylation. Cancer Lett. 255:42-48), and amaranth (Silva-Sánchez et al., 2008. Bioactive peptides in amaranth (Amaranthus hypochondriacus) seed. J. Agric. Food Chem. 56:1233-1240). The concentration of lunasin in soy can vary depending on the cultivar, culture pedoclimatic atmosphere and technological processes grains have been subjected to after the harvesting. Lunasin very high concentration has been found in Loda cultivar (about 11 mg/g), while in other soy varieties (for example. Imari) lunasin content does not exceed 5-6 mg/g (Wang et al. 2008. Analysis of soybean protein derived peptides and the effect of cultivar, environmental conditions, and processing of lunasin concentration in soybean and soy products. J. AOAC Intern. 91:936-944). Lunasin contains 9 aspartic acid residues at C-terminus of polypeptide chain. This composition favours an elevated affinity to hypo-acetylated chromatin regions, to which the peptide can bind thus inhibiting acetylation-deacetylation dynamics and, therefore, acting as tumour suppressor in carcinogenesis. It has been also reported that lunasin can exert a prevention activity against carcinogenesis phenomena, thanks to inhibition of cell proliferation induced by *ras* gene and to acetylation inhibition of H3 histone (Jeong et al., 2003. Characterization of lunasin isolated from soybean. J Agric Food Chem. 51: 7901-7906). From literature data it it is apparent that no study has considered up to now lunasin enrichment for soy derivatives by fermentation processes using lactic acid bacteria.

Based on above reported considerations, some elements appear to display a marked innovative character: (i) to employ soy based substrates, possibly of biological origin; (ii) to employ lactic acid bacteria isolated from food matrices and not of fecal origin; (iii) to optimize a biotechnological process suitable to favour the formulation of a preparation containing higher number of functional molecules such isoflavones-aglicones, equol and lunasin; (iv) to demonstrate, using in vitro and ex vivo assays, the effect of resulting preparation as to cutaneous and human intestinal cell protection, with particular reference to inhibition of inflammatory state and barrier function keeping.

The authors of the present invention now developed a new process suitable to provide a fermented soy based mixture enriched for isoflavones, aglicones, equol and lunasin. The mixture according to the invention is obtained by fermentation of soy using a particular mixture of lactic acid bacteria exclusively derived from food matrices and not of fecal origin. The mixture according to the invention, due to high content of isoflavones-aglicones, equol and lunasin, in particular lunasin, displayed particular effectiveness for cutaneous and human intestinal cell protection with particular reference to the prevention of inflammatory state and barrier function keeping.

The authors of the present invention have now considered that: (i) the selection of 103 isolated lactic acid bacteria, derived exclusively from food matrices, according to β-glucosidase activity on p-nitrophenyl-β-D-glucopyranoside (*pNPG*) allowed the selection of 4 lactic acid bacteria, namely *L. plantarum* DPPMA24W (deposited at DSMZ on 8 July 2010 DSM number 23756) and DPPMASL33 (deposited at DSMZ on 8 July 2010 DSM number 23755), *L. fermentum* DPPMA114 (deposited at DSMZ on 8 July 2010 DSM number 23757) and *L. rhamnosus* DPPMAAZ1 (deposited at DSMZ on 8 July 2010 DSM number 23758), to be used as mixed starter for fermentation of soy flour based substrates; (ii) the use of 14 different soy based substrates allowed to select as optimal the preparation based on organic farming soy flour for fermentation using mixed starter; (iii) the optimization of fermentation process of organic farming soy flour substrate allowed the formulation of a preparation comprising 1,45 mg/100 ml (57,0 µM) of daidzein, 3,9 mg/100 ml (140,3 µM) of genistein, 0,58 mg/100 ml (20,4 µM) of glycitein, 0,9 mg/100 ml (37.3 µM) of equol and 8,4 mg/100 ml of lunasin; (iv) the preparation based on above-mentioned functional compounds displays a protection effect on the cutaneous epidermis and positive effect on the inhibition of inflammatory state and barrier functions of intestinal cells.

Lactic acid bacteria according to the present invention belong to the *Lactobacillus* species and have been isolated from natural yeasts used for bread-making in Central and Southern Italy and from aged "pasta filata" cheeses of Pecorino type from Puglia region. Generally, the lactic acid bacteria isolated from such food matrices display metabolic and environmental adaptation characteristics not too much dissimilar than microorganisms colonizing gastrointestinal tract of humans and animals. *L*. *plantarum* DPPMA24W (deposited at DSMZ on 8 July 2010 DSM number 23756) and *L. plantarum* DPPMASL33 (deposited at DSMZ on 8 July 2010 DSM number 23755), *L. fermentum* DPPMA114 (deposited at DSMZ on 8 July 2010 DSM number 23757) and *L. rhamnosus* DPPMAAZ1 (deposited at DSMZ on 8 July 2010 DSM number 23758) have been selected and used.

A biotechnological protocol involving the fermentation by means said four lactic acid bacteria on various soy flour based substrates, preferably of biological origin for 48 - 96 h at 30 - 37°C has been standardized and optimized. At the end of fermentation process, cells can be removed or not from culture broth by means of centrifugation and subjecting the supernatant to a dehydration process by drying or freeze-drying.

Below biotechnological protocol for fermentation of the organic farming soy based preparation is described.

Propagation of selected 4 lactic acid bacteria cultures at 30°C for 24 h, washing, water suspension at a cellular density of 9,0 log cfu/ml and inoculum (1-4%) of soy milk(various soy flours, preferably organic farming soy)

| |
|---|
| ↓ |
| Culture at 30-37°C for 48 - 96 h |
| ↓ |
| Cell removal by centrifugation |
| ↓ |
| Supernatant dehydration by drying or freeze-drying |
| ↓ |
| Formulation of the preparation for medical applications |

As a result of fermentation of various soy milk preparations the synthesis of 3,9 - 57,0 µM of daidzein, 7,8 - 140,3 µM of genistein, 6,7 - 20,4 µM of glycitein, 7,6 - 37,3 µM of equol and about 8,4 mg/100 ml of lunasin has been obtained. Upper limits of above reported concentrations refer to fermentation of soy milk derived from organic farming soy flour. According to one of possible formulations, the application of fermented products from organic farming soy displayed to be suitable to: (i) protect epidermis enhancing barrier functions thereof; (ii) inhibit the inflammatory state of Caco-2/TC7 cells following the induction by γ-interpheron (IFN-γ) and lipopolysaccharide (LPS); (iii) stimulate barrier functions as demonstrated by Transepithelial Electric Resistance (TEER) test; and (iv) inhibit the synthesis of interleukin-8 (IL-8).

As demonstrated by complementary analysis using microbiological, chromatographic techniques and assays on in vitro and ex vivo cell cultures, the fermentation of organic farming soy flour by mixed starter consisting of lactic acid bacteria species isolated from food matrices and not used in previous studies, according to the present invention, allows: (i) the concomitant synthesis of aglicones, equol and lunasin, not found in previous studies and (ii) a protective effect against inflammatory state, enhancing the barrier function of epidermis and intestinal human cells.

It is therefore, a specific object of the present invention a process for the preparation of a fermented soy based mixture, comprising isoflavones-aglicones, equol and lunasin, by soy fermentation using a mixture of the following four lactic acid bacteria: *Lactobacillus plantarum* DSM 23755, *Lactobacillus plantarum* DSM 23756, *Lactobacillus fermentum* DSM 23757 and *Lactobacillus rhamnosus* DSM 23758. The mixture obtained according to the process of the invention is a mixture enriched for isoflavones-aglicones, lunasin, equol, that is it contains a greater percentage of these compounds in comparison to known mixtures obtained by processes using lactic acid bacteria different than those of the present invention.

The process according to the invention comprises or consists in the following steps:
a) culture propagating said four *Lactobacillus plantarum* DSM 23755, *Lactobacillus plantarum* DSM 23756, *Lactobacillus fermentum* DSM 23757 and *Lactobacillus rhamnosus* DSM 23758 lactic acid bacteria;
b) inoculating soy based substrates with an aqueous suspension of said lactic acid bacteria; preferably the substrates are inoculated with aqueous suspension of lactic acid bacteria in an amount from 1 to 4% of total substrate volume, said aqueous suspension having a cell density about log 9,0 cfu/ml for each biotype;
c) incubating at 30 - 37°C, preferably 30°C, for 48 - 96 h, preferably 96 h.

Soy based substrates suitable to be used are, for example, soy flour, preferably organic farming soy flour, soy milk and other commercial formulations as reported according to present application.

The process according to the invention can further comprise the step d) of centrifugation of broth-culture in order to separate cells from lactic acid bacteria. In particular, the centrifugation of the broth-culture can be carried out at 10.000 x *g* for 15 min at 4°C.

The process according to the present invention can further comprise a step e) of dehydration of supernatant obtained in step d) by drying or freeze-drying. According to an embodiment the formulation can contain viable, vital lactic acid bacteria omitting step d). The preparation of a composition can involve, at the end of the dehydration process, the formulation with addition of suitable excipients in order to obtain the preparation of forms suitable to the oral or topical use depending on circumstances.

It is a further object of the present invention a mixture, comprising isoflavones-aglicones, equol and lunasin, based on fermented soy obtainable according to the process as above defined without step d) of removal of lactic acid bacteria, said process optionally further comprising a step e) of dehydration of the culture obtained in step c) by drying or freeze-drying, wherein said mixture comprises the following four lactic acid bacteria: *Lactobacillus plantarum* DSM 23755, *Lactobacillus plantarum* DSM 23756, *Lactobacillus fermentum* DSM 23757 and *Lactobacillus rhamnosus* DSM 23758. Said mixture, therefore, contains above mentioned lactic acid bacteria according to the present invention. As above reported, the mixture according to the invention is a mixture enriched for isoflavones-aglicones, equol, and lunasin, that is, it contains an higher percentage of these compounds than known mixtures obtained by processes using lactic acid bacteria different than those of the present invention.

The present invention concerns, moreover, a pharmaceutical or cosmetic composition comprising or consisting of the mixture as above defined together with one or more pharmaceutically or cosmetically acceptable excipients and/or adjuvants.

According to a further embodiment, the mixture according to the invention can be used as a food integrator. For example the mixture could be used also for traditional foods, for example bake or pasta products.

Moreover, the mixture or the composition according to the invention can be used for the treatment of disorders or diseases of the skin or intestine. In particular, said mixture or composition can be used against modifications of skin barrier function, for example for prevention or treatment of sensitive skin, dried skin, psoriasis, atopic dermatitis, seborrheic dermatitis, dandruff, irritative dermatosis, eczema dermatosis, contact dermatosis, ulcers, acne, skin aging. Moreover, the mixture or composition according to the invention can be used in case of modification of intestinal barrier function, for example for the treatment or the prevention of the celiac disease, food intolerances, Crohn's disease.

The mixture or composition according to the invention can be used in cosmetic field, for example for treatment of hair loss or in medical field for the treatment of alopecia or telogen defluvium.

Particularly, the mixture or composition according to the invention can be administered by topical way, for example in form of creams, lotions, pastes, salves, gel, solutions, emulsions, suspensions or systemically, for example by oral way, for example as vial, chewable tablet, pill, sachet, etc.

Of course also the mixture obtained according to the process of invention comprising the step d) of removal of the lactic acid bacteria or a pharmaceutical or cosmetic composition containing the same can be advantageously used for above reported indications because it contains an higher percentage of isoflavones-aglicones, equol and lunasin, than known mixtures obtained by means of processes using lactic acid bacteria different than those of the present invention.

Moreover, a mixture of following four lactic acid bacteria, *Lactobacillus plantarum* DSM 23755, *Lactobacillus plantarum* DSM 23756, *Lactobacillus fermentum* DSM 23757 and *Lactobacillus rhamnosus* DSM 23758 is an object of the present invention. Finally *Lactobacillus plantarum* DSM 23755 or *Lactobacillus plantarum* DSM 23756 or *Lactobacillus fermentum* DSM 23757 or *Lactobacillus rhamnosus* DSM 23758 is an object of the present invention.

The present invention now will be described by an illustrative, but not limitative way, according to preferred embodiments thereof, with particular reference to enclosed drawings.

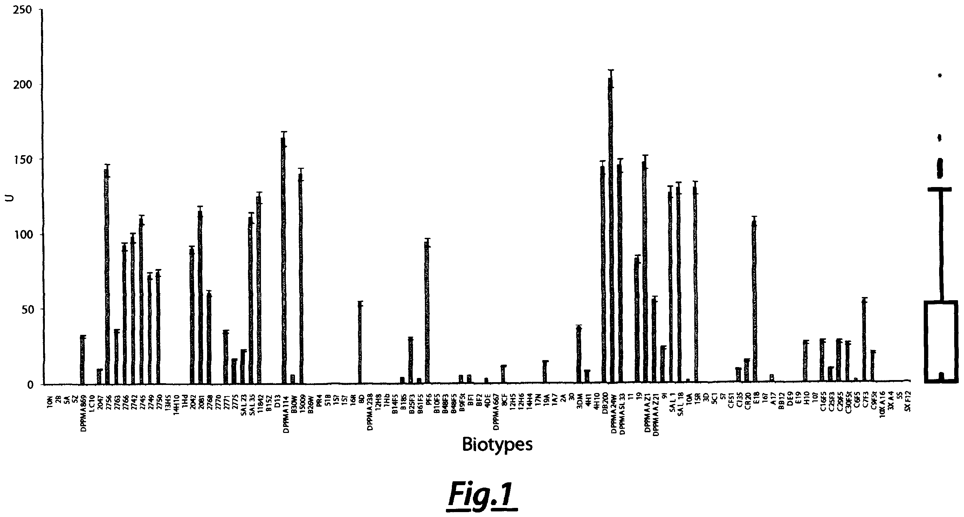

Figure 1 shows β-glucosidase activity of 103 lactic acid bacteria biotypes belonging to various species on *pNPG* synthetic substrate. All the lactic acid bacteria used in the assay have been previously isolated, in absolutely innovative way, only from food matrices. Lactic acid bacteria biotypes are indicated by code, in order to identify the correspondence thereof to the species, please refer to protocol description in the text (Example 1). Data are the average of three triplicate experiments. Statistical elaboration by box plot is reported

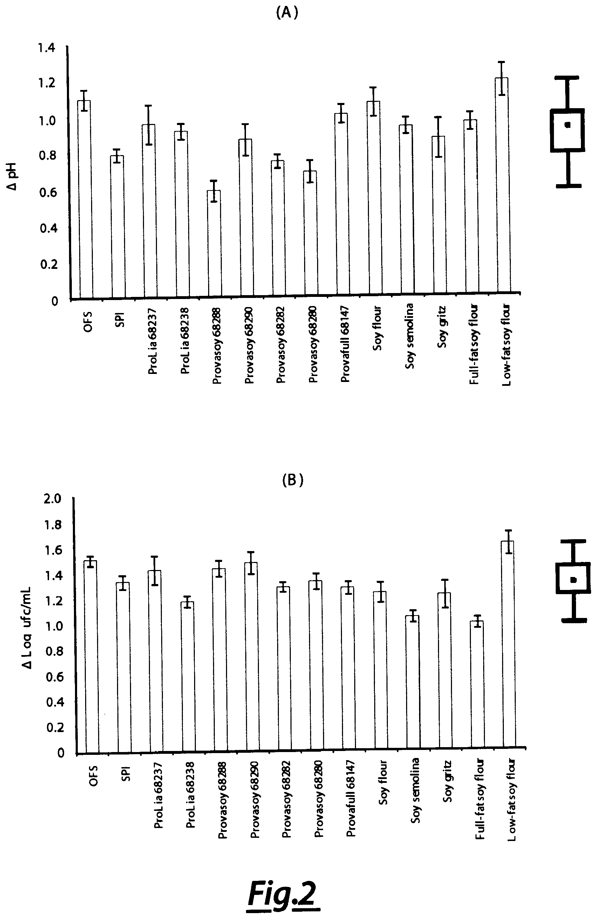

Figure 2A shows the lactic acidification process carried out by mixed starter selected in the presence of soy milk from 14 different flours. Figure 2B shows the cell density of lactic acid bacteria biotypes comprising the mixed starter selected in the presence of soy milk from 14 different flours. Data are the average of three triplicate experiments. Statistical elaboration by box plot is reported

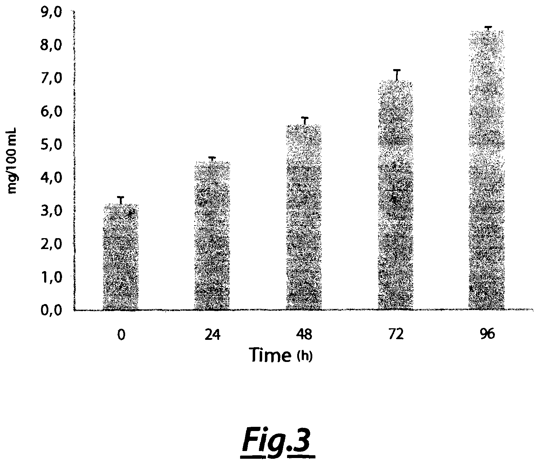

Figure 3 shows the synthesis of lunasin (mg/100 ml) during the fermentation of soy milk obtained from organic farming soy flour (OFS) using selected mixed starter. Data are the average of three triplicate experiments.

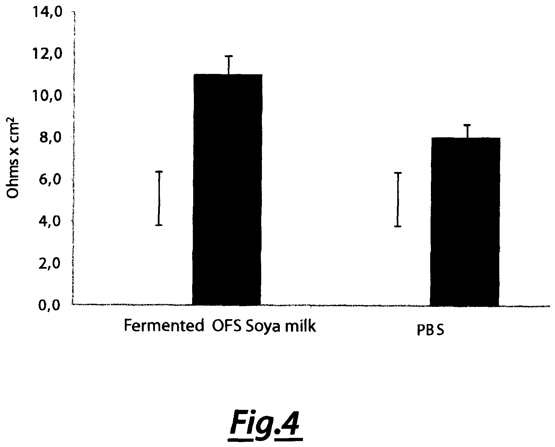

Figure 4 shows Transepithelial Electric Resistance (TEER) (Ohms x cm²) of reconstituted epidermis (SkinEthic®) after exposure for 0 and 24 h to fermented organic farming soy milk (OFS) using the selected mixed starter and PBS buffer. Data are the average of three triplicate experiments.

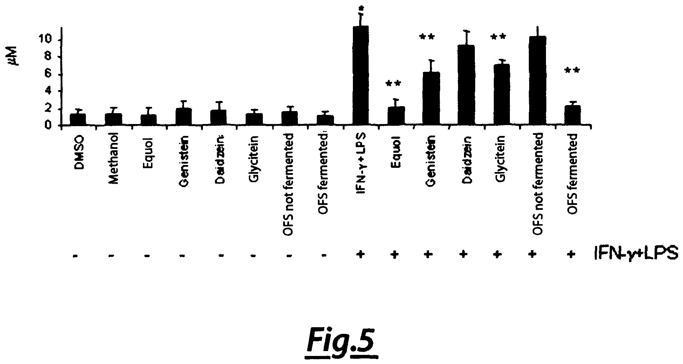

Figure 5 shows nitric oxide release (µM) (NO) from Caco-2/TC7 cells. The cells have been pre-treated for 24 h with chemical compounds (10 µM) used as standards (equol, daidzein, genistein and glycitein) and soy milk, obtained from organic farming soy flour, not fermented or fermented using mixed selected starter, diluted at equol final concentration of 10 µM and sterile filtered. Successively, the cells have been stimulated with γ-interpheron (IFN-γ) (1000 U/ml) and lipopolysaccharide (LPS) (100 ng/ml) for 24 h. DMEM culture medium containing DMSO (1%, v/v) or methanol (0.5%, v/v) has been used as negative control. Data are the average of three triplicate experiments. Asterisk indicates meaningful differences (*P<0.01*) compared to negative control.

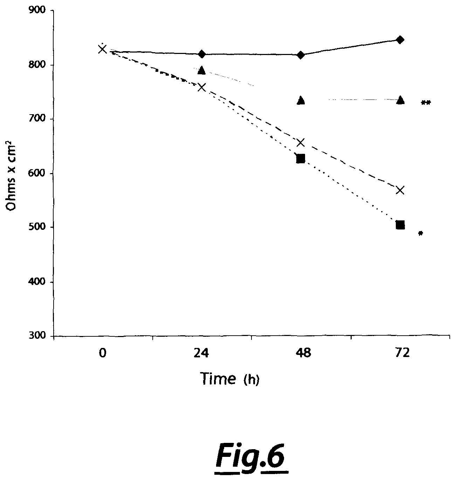

Figure 6 shows the Transepithelial Electric Resistance (TEER) (Ohms x cm²) of Caco-2/TC7 cells after 24, 48 and 72 h of incubation. The incubation has been carried out in the presence of γ-interpheron (IFN-γ) (1000 U/ml (- ■-); IFN-γ and soy milk, obtained from organic farming soy flour, not fermented (- ▲ -) or fermented with the selected mixed starter, diluted at equol final concentration of 10 µM and sterile filtered. (-x-). DMEM culture medium has been used as negative control (- ◆-). Data are the average of three triplicate experiments. Asterisk indicates meaningful differences (*P<0.01*) compared to negative control.

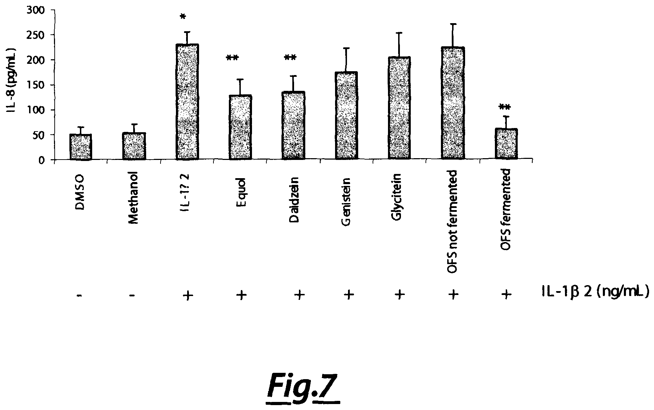

Figure 7 shows the release (pg/ml) of interleukin-8 (IL-8) from Caco-2/TC7 cells stimulated for 24 h with interleukin-1β (IL-1β) (2 ng/ml) and successively treated (24 h) with chemical compounds (10 µM) used as standards (equol, daidzein, genistein and glycitein) and with soy milk, obtained from organic farming soy flour, not fermented or fermented with the selected mixed starter, diluted at equol final concentration of 10 µM and sterile filtered. DMEM culture medium containing DMSO (1%, v/v) or methanol (0.5%, v/v) has been used as negative control. Data are the average of three triplicate experiments. Asterisk indicates meaningful differences (*P<0.01*) compared to negative control.

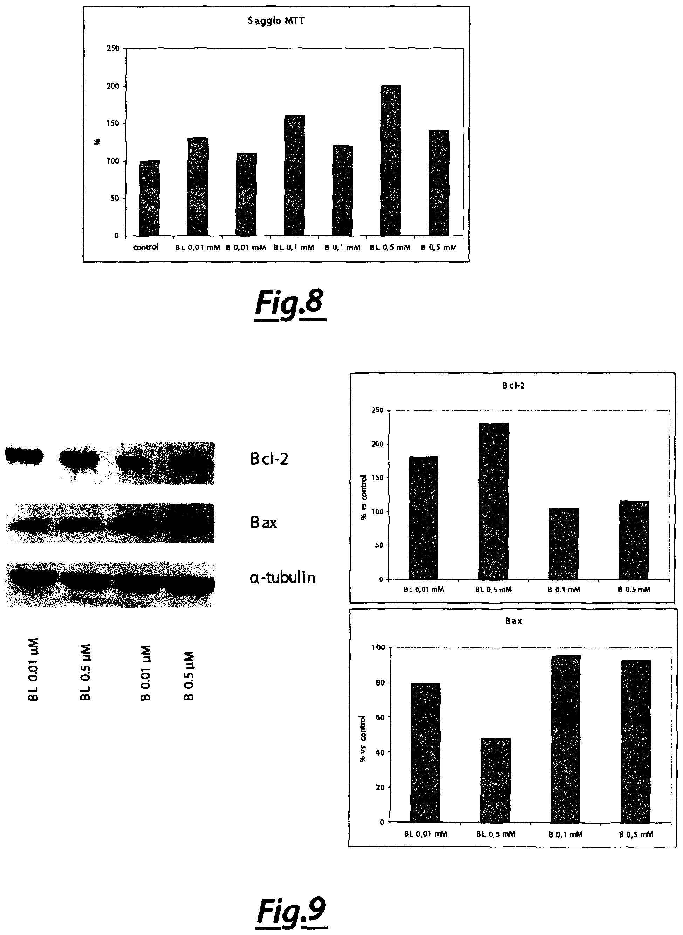

Figure 8 shows the effect of biomass containing lunasin and without lunasin on the cell proliferation.
Figure 9 shows the effect of the biomass containing lunasin and without lunasin on the protein expression of Bcl-2 and Bax.

### Example 1: β-glucosidase activity of 103 biotypes of lactic acid bacteria isolated from food matrices

One hundred and three biotypes of lactic acid bacteria belonging to *Lactobacillus alimentarius* (10N, 2B, 5A), *Lactobacillus brevis* (5Z, DPPMA869), *Lactobacillus casei* (LC10), *Lactobacillus casei* subsp. *casei* (2047, 2756, 2763, 2766), *Lactobacillus casei* subsp. *pseudoplantarum* (2742, 2745, 2749, 2750), *Lactobacillus curvatus* (13H5, 14H10, 1Hd, 2042, 2081, 2768, 2770, 2771, 2775, SAL23, SAL35), *Lactobacillus delbrueckii* subsp. *bulgaricus* (11842, B₁₅Z), *Lactobacillus fermentum* (DPPMA114, D13), *Lactobacillus gasseri* (B₃₀W), *Lactobacillus helveticus* (15009, B₂₆W, PR4), *Lactobacillus hilgardii* (51 B), *Lactobacillus paralimentarius* (15α, 15β, 16R, 8D, DPPMA238), *Lactobacillus paracasei* (12H8, 1Hb, B₁₄F₅, B₁₈S, B₂₅F₃, PF6, B₆₁F₅), *Lactobacillus pacarbuckneri* (B₁₀F₅, B₄₈F₃, B₄₈F₅, B₉F₅ₜ, BF₁, BF₂), *Lactobacillus paraplantarum* (4DE, DPPMA667), *Lactobacillus pentosus* (8CF, 12H5, 12H6), *Lactobacillus plantarum* (14H4, 17N, 19A, 1A7, 2A, 30, 3DM, 4H1, 4H10, DB200, DPPMASL33, DPPMA24W), *Lactobacillus rhamnosus* (11, 19, DPPMAAZ1, DPPMAAZ21), *Lactobacillus sakei* (91, SAL1, SAL18), *Lactobacillus rossiae* (10A, 15R, 3D, 5C1, 5α, CF51, Cl35, CR20, E18), *Lactobacillus sanfranciscensis* (16α, A17, BB12, DE9, E19, H10), *Lactococcus lactis* subsp. *lactis* (10γ), *Pediococcus pentosaceus* (C₁₆F₅, C₂₅F₃, C₃₀F₅ₜ, C₆F₅, C₇F₃, C₉F₅ₜ, C₂₉F₅) and *Weissella cibaria* (10XA16, 3XA4, 5S, 5XF12) species have been used in the present study. All the biotypes belong to the Collezione di Colture del Dipartimento di Protezione delle Piante e Microbiologia Applicata dell'Università degli Studi di Bari, and have been previously isolated from food matrices (natural yeast for bread-making and cheeses). Biotypes of lactic acid bacteria have been propagated at 30°C for 24 h in MRS medium (Oxoid, Basingstoke, United Kingdom) at 30 or 37°C for 24 h.

Cells cultured for 24 h, collected by centrifugation (10,000 x *g* for 15 min at 4°C), washed two times with phosphate buffer 50 mM, pH 7,0 and re-suspended in water at cell density of log 9,0 cfu/ml have been used for β-glucosidase activity assay. β-glucosidase activity has been quantified as *p-nitrophenol* released from p-nitrophenol-β-D-glucopyranoside (*pNPG*) substrate (Sigma Aldrich Chemical Corporate, St.Louis, Missouri, USA). 900 µl of *pNPG* (final concentration) in phosphate buffer 0,5 M, pH 7,5, and 100 µl of cell suspension have been used for assay. The mixture has been incubated at 40°C and the reaction blocked by heat treatment at 95°C for 5 min. Absorbance has been measured at 410 nm. One β-glucosidase unit (U) activity has been defined as the enzyme amount needed in order 1 nmol/min of p-nitrophenol to be released under assay conditions (De Angelis et al., 2005. Purification and characterization of an intracellular family 3 β-glucosidase from Lactobacillus sanfranciscensis CB1. Ital. J. Food Sci. 17:131-142).).

### Example 2: Preparation and fermentation of soy milk

Organic farming soy (organic farming soybean, OFS) (ECorNaturaSi, Verona, Italy), soy protein isolate (SPI) (Supro Soja 80 Aptonia, Villeneuve d' Ascq, France) and various commercial preparations of soy flours (Cargill Texturizing Solutions Soy Protein, Gent, Belgium) have been used for production of soy milk. OFS has been washed and left standing over night in distilled water. Wet and swollen soy has been manually decorticated, diluted with warm water (about 90°C), at 1:10 ratio, and homogenised with PBI International homogeniser (Milan, Italy). The homogenization has been carried out at 10,000 x *g* for 2 min, followed by 1 min pause and again treated at 14.000 x *g* for 2 min. The suspension has been centrifuged (7,000 x *g,* 10 min at 4°C) and soy milk sterile filtered through 0,22 µm pore size filter (Millipore Corporation, Bedford). The pH was 6,2. SPI has been diluted with distilled water (40°C), at 0,4:10 ratio, and thermally treated at about 55°C for 30 min under stirring (120 rpm). After cooling at room temp., the pH was adjusted at 6,7 using NaOH 5 M (Tsangalis et al. 2002). Sterilization has been carried out in autoclave at 121°C for 15 min. The commercial soy flour preparations have been diluted with distilled water (40°C), at 1:10 ratio, according to method described by Chun et al. (Chun et al., 2007. Conversion of isoflavone glucoside to aglycones in soymilk by fermentation with lactic acid bacteria. J. Food Sci. 72:39-44). pH value was about. 6,3. Sterilization has been carried out in autoclave at 121°C for 15 min.

Different soy milk types have been inoculated (1 - 4%, v/v) with a mixed cell suspension of 4 lactic acid bacteria selected on the basis of β-glucosidase activity. Initial cell density of each 4 lactic acid bacteria biotypes was log 7,0 cfu/ml. Fermentation has been carried out at 30°C for 96 h under stirring (120 rpm). For human intestinal cell assay, soy milk has been frozen-dried, re-suspended in DMEM culture medium and sterile filtered.

### Example 3: Monitoring of the lactic acid bacteria, determination of isoflavones-aglicones, equol and lunasin

The monitoring of lactic acid bacteria used as mixed starter (*Lactobacillus plantarum* DSM 23755 corresponding to DPPMASL33, *Lactobacillus plantarum* DSM 23756 corresponding to DPPMA24W, *Lactobacillus fermentum* DSM 23757 corresponding to DPPMA114 and *Lactobacillus rhamnosus* DSM 23758 corresponding to DPPMAAZ1) during the fermentation of the various soy milk types has been carried out using RAPD-PCR technique. Two primers (Invitrogen, Milan, Italy), with arbitrarily selected sequences (P7 5' AGCAGCGTGG 3'(SEQ ID No:1), and M13, 5' GAGGGTGGCGGTTCT 3' (SEQ ID NO:2)), randomly amplifying different regions of plasmid and chromosomal bacterial DNA (De Angelis et al., 2001. Characterization of non-starter lactic acid bacteria (NSLAB) from ewes' Italian cheeses based on phenotypic, genotypic and cell-wall protein analyses. Appl. Environ. Microbiol. 67:2011-2020; Rossetti e Giraffa, 2005. Rapid identification of dairy lactic acid bacteria by M13-generated, RAPD-PCR fingerprint databases. J. Microbiol. Met. 63:135-144) have been used fortypizing.

The extraction of isoflavones-aglicones and equol from fermented soy milk samples has been carried out according to method described by Otieno and Shah (Otieno and Shah, 2007. A comparison of changes in the transformation of isoflavones in soymilk using varying concentrations of exogenous and prebiotic-derived endogenous β-glucosidases. J. Appl. Microbiol. 103:601-612). HPLC chromatographic analysis for the determination of compounds has been carried out according to method described by Maubach et al. (Maubach et al., 2003. Quantitation of soy-derived phytoestrogens in human breast tissue and biological fluids by high-performance liquid chromatography. J. Chromatogr. 784:137-144).

The determination of lunasin in soy milk obtained from organic farming soy flour before and during the fermentation has been carried out by HPLC chromatography using an AKTA Purifier system (GE Healthcare) equipped with C18 Xterra Waters column and 214 nm UV detector, eluting with mixture solvent consisting of 5% ACN + 0.05% TFA (eluent A) and ACN + 0.05% TFA (eluent B) (Wang et al. 2008. Analysis of soybean protein derived peptides and the effect of cultivar, environmental conditions, and processing of lunasin concentration in soybean and soy products. J. AOAC Intern. 91:936-944). Synthetic lunasin has been used as standard (EZBiolab Inc., Carmel, IN, USA).

### Example 4: Tests on reconstituted epidermis and TEER measurement (Transepithelial Electric Resistance)

Human reconstituted epidermis SkinEthic® (Reconstructed Human Epidermis) consists of normal keratinocytes of human epidermis as a multilayer. It is completely differentiated epidermis after culture of human keratinocytes in a chemically defined medium (MCDM 153), without bovine foetal serum addition, on inert porous polycarbonate support at air-liquid interface for 17 days. At this growth stage the morphologic analysis shows a vital multi-stratified epidermis and a corneous layer consisting of more than ten compact cellular layers. Human reconstituted epidermis SkinEthic® has been used according to previously described procedures (Di Cagno et al., 2009. Synthesis of γ-amino butyric acid (GABA) by Lactobacillus plantarum DSMZ19463: functional grape must beverage and dermatological application. Appl Biotechnol Microbiol DOI: 10.1007/s00253-009-23704)..

TEER measurement has been executed using Millicell-ERS Volthommeter (Millipore, Billerica, MA). Measurement has been expressed in Ohms x cm².

### Example 5: tests on Caco-2/TC7 cells

Human origin Caco-2 cells (clone TC7) have been cultured in Dulbecco (DMEM) medium, added with bovine foetal serum (10%), not essential amino acids (1%), gentamycin/streptomycin (50 µg/ml), glutamine (2 mM) and 4-2-hydroxyethyl-1-piperazinyl-ethanesulfonic acid (1%) (Di Cagno et al., 2010. Quorum sensing in sourdough Lactobacillus plantarum DC400: induction of plantaricin A (PlnA) under co-cultivation with other lactic acid bacteria and effect of PlnA on bacterial and Caco-2 cells. Proteomics in press). The cells viability has been determined by uptake assay of Neutral Red dye (Balls et al., 1987. Approaches to validation alternative methods in toxicology. In: Goldber A.M. (Ed). N.Y. Academic Press pp. 45-58). After treatment for 24 - 72 h with the different preparations, the cells have been washed with PBS buffer and incubated for 4 h at 37°C with Neutral Red solution (33 mg/l). Then the cells have been washed again with PBS buffer and treated with lysis solution (50 % ethanol in water containing 1% acetic acid). Plate reading has been carried out using Novapath reader (Biorad, Hercules, CA) (Di Cagno et al., 2010).

The nitric oxide release (NO) from Caco-2/TC7 cells has been determined by measuring the oxidation products, i.e. nitrite and nitrate. After incubation with the different preparations, the supernatant of cell cultures has been mixed with an equal volume of Griess reagent (1%, p/v, sulfanilic acid in 0,5 M HCl and 0.1%, p/v, N-1-naphthylethylendiamine hydrochloride). After 30 min of incubation at room temp., the absorbance at 540 nm has been measured Nitrite concentration has been determined with reference to standard curve prepared with sodium nitrite.

For TEER measurement Caco-2/TC7 cells have been inoculated (7,5 x 10⁴ cell/ml) in a microplate container with 24 cells and a polyethylene filter (0,4 µm pore size). Before the treatment, the cells have been incubated for 21 days at 37°C. The treatments with various preparations have been carried out for 18, 24 and 48 h. Integrity of cellular layer then has been determined by means of TEER measurements.

For measurement of released interleukin-8 (IL-8) Caco-2/TC7 cells have previously been incubated (24 h) with interleukin-1β and then stimulated for further 24 h with the different preparations. The synthesis of pro-inflammatory IL-8 has been determined by ELISA assay (Bender MedSystems). The quantification has been carried out using a standard curve according to .kit instructions

### Results

### (1) Selection of lactic acid bacteria on the base of β-glucosidase activity

Preliminarily, β-glucosidase activity of 103 biotypes of lactic acid bacteria isolated from food matrices has been tested on *pNPG* synthetic substrate. The activity changed from 0 to 202,3 U (Figure 1). Forty-eight biotypes belonging mostly to *L. alimentarius, L. brevis, L. casei, L. delbrueckii* subsp. *bulgaricus, L. helveticus, L. hilgardiii, L. paralimentarius, L. paraplantarum, L. pentosus, L. sanfranciscensis, Lc. lactis* subsp. *lactis, L. parabuchneri* e *W. cibaria* species did not displayed β-glucosidase activity. The activity average value was 3 U, and values corresponding to 25° and 75° data percentile were 0 and 45,5 U. Twenty-five biotypes belonging to different species of lactic acid bacteria have displayed β-glucosidase activity higher than 55 U. *L. plantarum* DPPMA24W, *L. fermentum* DPPMA114, *L. rhamnosus* DPPMAAZ1 and *L*. *plantarum* DPPMASL33 have displayed higher activities (202,35 ± 7,08, 163,15 ± 6,52, 146,60 ± 5,84 and 144,34 ± 7,19 U, respectively). The values of β-glucosidase activity for these biotypes have been out of box plot error bar. Based on these result the four lactic acid bacteria have been selected and used for the formulation of a mixed starter to be used for fermentation of the various soy milk types.

### (2) Fermentation of soy milk and synthesis of functional compounds

The chemical composition, protein dispersibility index and particle size of various soy flour types used for the preparation of soy milk are reported in Table 1. Table 1 shows the chemical composition, protein dispersibility and particle size of 14 soy flours used for functional compound production by selected mixed starter comprising *Lactobacillus plantarum* DSM 23755 corresponding to DPPMASL33, *Lactobacillus plantarum* DSM 23756 corresponding to DPPMA24W, *Lactobacillus fermentum* DSM 23757 corresponding to DPPMA114 and *Lactobacillus rhamnosus* DSM 23758 corresponding to DPPMAAZ1.

| **Table 1.** Chemical composition, protein dispersibility index and granulometry of commercial soy flours | | | | | | |
|---|---|---|---|---|---|---|
| Soy Flour | Proteins (%) | Lipids (%) | Fibers (%) | Protein dispersibility | Granulometry (mesh) * | Description |
| OFS** | 13,1 | 6,7 | 1,1 | 65,3 | 160 | Manually decorticated |
| SPI*** | 83 | 4,4 | 1,5 | 20,1 | 72 | Protein isolated from soy, extract, aspartame |
| Prolia 68237 | 54 | 0,95 | 3,5 | 70 | 200 | De-fatted, mild heat |
| Prolia 68238 | 55 | 1,1 | 3,5 | 77,5 | 200 | De-fatted, not toasted |
| Provasoy 68288 | 54 | 1,25 | 3,5 | 22,5 | 100 | De-fatted, enzymatically |
| Provasoy 68290 | 54 | 1,25 | 3,5 | 22,5 | 200 | De-fatted, enzymatically |
| Provasoy 68282 | 54,2 | 1,0 | 3,5 | 22,5 | 100 | De-fatted, enzymatically |
| Provafull 8147 | 39,0 | 21,0 | 3,5 | 10,3 | 72 | Toasted |
| Soy flour | 40 | 2 | 20 | 21,6 | 120 | Toasted |
| Soy semolina | 38 | 22 | 12,4 | 18,6 | 120 | Decorticated and toasted |
| Soy gritz | 38 | 22 | 20 | 16,1 | 11 | Decorticated and toasted |
| Full-fat soy flour | 38,2 | 23 | 16,7 | 19,3 | 100 | Mechanically decorticated |
| Low-fat soy flour | 45,6 | 11,7 | 18,2 | 20,1 | 100 | Extruded, mechanically |

| | | | | | | |
|---|---|---|---|---|---|---|
| *Mesh, mesh/pound;**OFS, organic farming soy;***SPI, soy protein isolate | | | | | | |

Fourteen soy milk types have been fermented using selected mixed starter comprising *L. plantarum* DPPMA24W and DPPMASL33, *L*. *fermentum* DPPMA114 and *L. rhamnosus* DPPMAAZ1. All the substrates have been subjected to a process of lactic acidification (Figure 2A). After 96 h of fermentation, ΔpH values changed from 0,59 ± 0,06 to 1,19 ± 0,09, for soy milk types obtained from Provasoy 68288 and Low-fat soy flours, respectively. ΔpH average value was 0,93, and the range corresponding to 25° and 75° data percentile value was 0,79 and 1,01. After fermentation, pH values for all soy milk types were within 5.1 - 5,3 range.

Lactic acid bacteria grew during the fermentation of all soy milk types (Figure 2B). Δlog cfu/ml values changed from 0,99 ± 0,29 to 1,61 ± 0,30, for soy milk types from Full-fat and Low-fat soy flours, respectively. Average value of cell density increase was 1,31 Δlog cfu/ml, corresponding to cell density absolute value of log 8.31 cfu/ml. Range corresponding to 25° and 75° percentile data value was 1.21 and 1,43. Growth of lactic acid bacteria was complete over 24 - 36 h of incubation. As determined by RAPD-PCR typizing, all four biotypes of lactic acid bacteria used in mixed starter grew on various soy milk types up a similar cell density.

Initial concentration of conjugated isoflavones in various soy milk types changed from 142,3 ± 12,5 to 171,5 ± 10,8, 102,2 ± 8,6 to 123,0 ± 11,3, and from 10,5 ± 1,1 to 18,0 ± 0,9 mM, respectively for daidzin, genistin and glycitin. On the contrary, low concentrations (from 0 to 7,8 ± 0,5 µM) of aglicones have been observed in various soy milk types (Table 2). Table 2 shows the concentration (µM) of isoflavones-aglicones (daidzein, genistein and glycitein) and equol during the fermentation of soy milks, obtained from 14 various flour types, using selected mixed starter. Data are the average of three triplicate experiments.

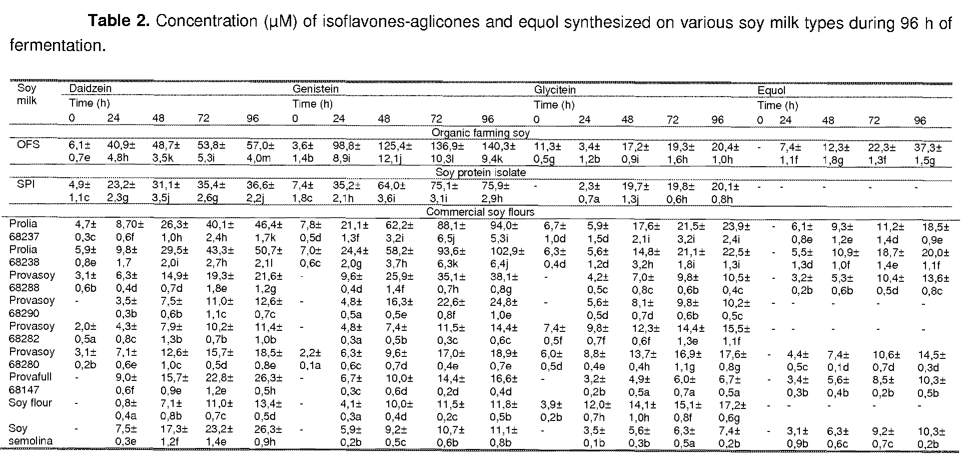

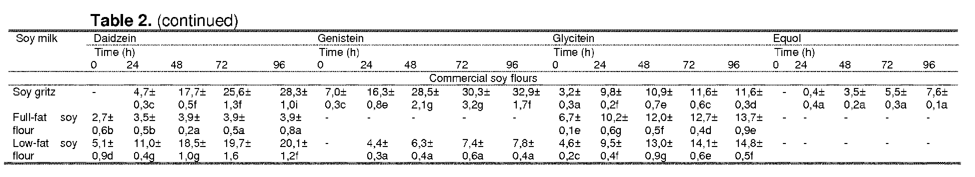

With the exception of soy milk obtained from Full-fat soy flour, the concentration of aglicones increased during the incubation for all soy milk types. After 96 h of incubation, the highest concentration of daidzein has been observed in OFS soy milk (57,0 ± 4,0 µM, corresponding to 1,45 mg/100 ml), followed by Prolia 68238 (50,7 ± 2.1 µM) and Prolia 68237 (46,4 ± 1,7 µM). Also final highest concentration of genistein was in above said three soy milk types (140,3 ± 9,4 - 3,9 mg/100 ml, 102,9 ± 6,4 and 94,0 ± 5,3 µM, for OFS, Prolia 68238 and 68237, respectively). Compared to others aglicones, the glycitein concentration was lower in all soy milk types. Highest concentration of glycitein was in Prolia 68237 and 68238, and OFS soy milk types (23,9 ± 2,4, 22,5 ± 1,3 and 20,4 ± 1,0 µM - 0,58 mg/100 ml, respectively). In the case of soy milk produced from organic farming soy flour (OFS) the conversion rate of conjugated isoflavones to corresponding aglicones was 0,72, 0,85 and 0,98, for daidzin to daidzein, genistin to genistein and glycitin to glycitein. Although the concentration of aglicones increased during the incubation, after 24 h hydrolysis rates of all three conjugated isoflavones were in 1,0 - 0,95 range.

Before the incubation, the presence of equol has not been detected in no type of soy milk (Table 2). Various soy milk types were unable to synthesise equol during fermentation process. After 96 h of incubation, the highest concentration of equol has been determined in Prolia 68238 and 68237 soy milk types (20,0 ± 1.1 and 18,5 ± 0,9 µM, respectively) and above all in OFS soy milk (37,3 ± 1,5 µM, corresponding to 0,9 mg/100 ml). Due to the simultaneous synthesis of daidzein, it was not possible to determine the conversion rate thereof to equol. Various studies have considered the use of potentially probiotic bacteria, isolated from human fecal material, in order to enrich soy milk with isoflavones-aglicones (Chun et al., 2007. Conversion of isoflavone glucoside to aglycones in soymilk by fermentation with lactic acid bacteria. J. Food Sci. 72:39-44; Donkor and Shah 2008. Production of β-glucosidase and hydrolysis of isoflavone phytoestrogens by Lactobacillus acidophilus, Bifidobacterium lactis and Lactobacillus casei in soymilk. J. Food Sci. 73:15-20; Pham and Shah 2007. Biotransformation of isoflavone glycosides by Bifidobacterium animalis in soymilk supplemented with skim milk powder. J. Food Sci. 72:316-324; Tsangalis et al., 2002; Tsangalis et al., 2004; Wei et al., 2007. Using Lactobacillus and Bifidobacterium to product the isoflavone algycones in fermented soymilk. Int. J. Food Microbiol.117:120-124). Used microorganisms have been exclusively bifidobacteria or various lactic acid bacteria belonging to various species. The present invention has selected four new biotypes corresponding to *L*. *plantarum* DPPMA24W and DPPMASL33, *L. fermentum* DPPMA114 and *L. rhamnosus* DPPMAAZ1, never used previously for the synthesis of isoflavones-aglicones and equol. Only a limited number of studies has considered also the synthesis of equol during the fermentation of soy milk. Equol has been synthesized in soy milk fermented with bifidobacteria (Tsangalis et al., 2002. Enzymatic transformation of isoflavone phytoestrogens in soymilk by β-glucosidase producing bifidobacteria. Food Res. Int. Sci. 67:3104-3113). After 24 h of fermentation, highest concentration of equol (0.521 mg/100 ml) was synthesized by *Bifidobacterium animalis,* compared with the production of 0,338 and 0,433 mg/100 ml obtained using *Bifidobacterium pseudolongum* and *Bifidobacterium longum* biotypes. OFS soy milk fermented with the mixed starter selected according to this study contained higher concentration of equol, namely 37,3 µM corresponding to 0,9 mg/100 ml.

Based on previously reported results, soy milk produced from organic farming soy flour (OFS) has been considered the best substrate for the synthesis of isoflavones-aglicones and equol. On the base of our knowledge, no previous study considered the use of soy milk obtained from biologically cultured soy flour for the synthesis of isoflavones-aglicones and equol.

Therefore, the concentration of lunasin using HPLC method (Wang et al. 2008. Analysis of soybean protein derived peptides and the effect of cultivar, environmental conditions, and processing of lunasin concentration in soybean and soy products. J. AOAC Intern. 91:936-944) has been determined. Before the incubation, the lunasin concentration was about. 3,2 mg/100 ml (Figure 3). During the fermentation, the selected mixed starter favoured a constant increment of lunasin that, at the end of 96 h of incubation, was about 8,4 mg/100 ml. On the base of our knowledge, no previous study considered the concomitant synthesis of isoflavones-aglicones, equol and lunasin in the same preparation consisting of soy milk fermented with lactic acid bacteria. The physiological effects of this bioactive peptide (lunasin) are widely documented in literature (Jeong et al., 2003. Characterization of lunasin isolated from soybean. J Agric Food Chem. 51: 7901-7906; Jeong et al. 2007. The cancer preventive peptide lunasin from wheat inhibits core histone acetylation. Cancer Lett. 255:42-48).

Based on previous results, fermented OFS soy milk was used for assays of cutaneous protection and on intestinal human cells.

### (3) Tests on reconstituted epidermis and TEER measurement (Transepithelial Electric Resistance)

OFS soy milk obtained from organic farming soy flour and fermented with selected mixed starter have been used at equol final concentration of 10 µM for treatment of human reconstituted epidermis according to the SkinEthic® model. This model has been wide experimented and accepted by the scientific community (Di Cagno et al., 2009. Synthesis of γ-amino butyric acid (GABA) by Lactobacillus plantarum DSMZ19463: functional grape must beverage and dermatological application. Appl Biotechnol Microbiol DOI: 10.1007/s00253-009-23704). After treatment for 24 h, TEER measurement has been carried out. This type of analysis, widely accepted by the international scientific community, evaluates the corrosion capacity of tissue taking as a reference the integrity of corneous layer and the barrier function. Particularly, by means of this detection it is possible to obtain information about the presence of a compact lamellar structure at corneous layer level, of integral tight junctions and epidermic thickness. These factors as a whole define an efficient barrier function. Figure 4 shows as in the presence of fermented OFS soy milk a remarkablel increase (*P<0,05*) of TEER value is present, demonstrating a protecting action of the molecule at cutaneous level. The same result has been obtained with a mixture of chemically synthesised equol and lunasin.

According to current knowledge this is the first application example of preparation based on soy milk containing isoflavones-aglicones, equol and lunasin demonstrating a stimulation of the cutaneous barrier functions.

### (4) Tests on Caco-2/TC7cells

With the purpose to test immunomodulating properties of isoflavones-aglicones contained in soy milk produced from organic farming soy flour (OFS), cytotoxicity against Caco-2/TC7 cells by standard chemical compounds (equol, daidzein, genistein and glycitein) at concentrations of 5 - 100 µM using Neutral Red (NR) uptake assay, firstly has been evaluated. Genistein, glycitein and equol have shown a behaviour similar to methanol and DMSO (negative control) and did not affect significantly cell proliferation. After 72 h of treatment, daidzein remarkably inhibited (P < 0,03) cell proliferation at concentration higher than 100 µM.

Preliminarily, Caco-2/TC7 cells have been treated for 24 h at concentration of 10 µM with the OFS fermented soy milk and diluted at equol final concentration of 10 µM or with not fermented soy milk. These compounds or preparations did not display induction for NO release, showing a behaviour similar to negative control, i.e. methanol and DMSO (Figure 5). Successively, Caco-2/TC7 cells have been stimulated with INF-γ(1000 U/ml) e LPS (100 ng/ml) per 24 h. This treatment significantly increased (*P* < 0,05) the NO release, thus simulating the inflammatory state of Caco-2/TC7 cells, preventively treated with negative control, daidzein or not fermented OFS soy milk. On the contrary, treatments with equol or fermented OFS soy milk inhibited in a marked manner (*P* < 0,002 and *P* < 0,007, respectively) the NO release. A considerable inhibition of NO release has been also observed using treatments with genistein and glycitein (*P* < 0,05). Since preliminarily it has been demonstrated that concentration (10 µM) of isoflavones-aglicones used in the test is not toxic, the death of Caco-2/TC7 cells has not surely interfered with NO release.

Under culture conditions of this study, Caco-2/TC7 cells develop morphological and functional characteristics of enterocytes, including tight intercellular junctions, integrity thereof being measured by TEER determination. Preliminarily, TEER has been determined in the presence of standard chemical compounds (10-100 µM), fermented OFS soy milk and diluted at equol concentration of 10 µM, or not fermented OFS soy milk. With the exception of equol chemical compound at concentration of 100 µM (1000 U/ml) effects on TEER during 72 h of incubation have not been observed. Treatments of Caco-2/TC7 cells with INF-γ(1000 U/ml) favoured a remarkable decrease (*P* < 0,003) of TEER value (Figure 6). When Caco-2/TC7 cells stimulated with INF-γ have been treated also with fermented OFS soy milk the negative effect of INF-γ is remarkably attenuated (*P* < 0,007). A negligible effect has been observed in the presence of not fermented OFS soy milk. Genistein, glycitein and above all equol have shown a trend similar to fermented OFS soy milk. Daidzein has not interfered with the negative effect caused by INF-γ.

Interleukin-8 (IL-8) is a member of C-X-C chemokine family and plays a fundamental role in activation of neutrophil cells, thus initiating the inflammatory response. When Caco-2/TC7 cells are subjected to a treatment with inteleukin-1 β (2 ng/ml) has been observed a meaningful increment (*P* < 0.001) of IL-8 synthesis (Figure 7). When Caco-2/TC7 cells, stimulated with interleukin-1 β, have been subjected also to a treatment with equol and daidzein a meaningful decrement (*P* < 0,005) of IL-8 synthesis has been observed. Highest inhibition of IL-8 synthesis (*P* < 0.001) has been observed by treatment with fermented OFS soy milk. On the contrary, treatments with genistein, glycitein or OFS soy milk fermented did not resulted in (*P* < 0,10) a decrement of IL-8 synthesis.

Reported results clearly show that the anti-inflammatory and stimulating effect to barrier functions of intestinal human cells by fermented OFS soy milk is mainly the result of the presence of equol and some isoflavones-aglicones. An additive effect by lunasin is possible.

### (5) Development a biotechnological protocol for the synthesis of daidzein, genistein, glycitein, equol and lunasin and use thereof in dermatological field

As previously outlined in other part of the text, a biotechnological process for the synthesis of isoflavones-aglicones (daidzein, genistein and glycitein), equol and lunasin and use thereof in dermatological field has developed. Said process involves:
a) culture of *L. plantarum* DPPMA24W and DPPMASL33, *L*. *fermentum* DPPMA114 and *L. rhamnosus* DPPMAAZ1 in pure culture on MRS culture medium;
b) collection, washing and inoculum of the cell suspensions in various soy milk types, preferably, sterile soy milk prepared from soy flour cultured according to agronomic biological methods and laboratory decorticated;
c) fermentation of soy milk by selected mixed starter for 48 - 96 h, preferably 96 h at 30 - 37°C, preferably 30°C;
d) separation of cells by centrifugation. According to a process variant the preparation can also contain lactic acid bacteria cells;
e) dehydration of the preparation by drying or freeze-drying process;
f) preparation of a composition by addition of suitable excipients in order to obtain forms suitable to use by oral or topical administration depending on the cases.

### Example 6: In vitro evaluation of biomass containing lunasin vs biomass without lunasin on stimulation of hair growth.

In vitro study of biomass containing lunasin (BL) compared to without lunasin (B) as promoter for hair growth.

### Material and methods

Derma papilla cells (DPCs) have been cultured in medium (Dulbecco' s modified Eagle' s medium, DMEM) containing 2 mM L-glutamine, 1x of antimycotic and antibiotic solution (1000u g/ml streptomycin sulfate, 1000 unit/ml penicillin G and 2,5 µg/ml amphotericin B) and 10% bovine foetal serum. At confluence the cells have been cultured for 24 hours in DMEM without serum and then treated with various concentrations of biomass containing or not lunasin.

The cell proliferation has been determined by MTT method (Mosmann, 1983). DPCs have been seeded in a 96 well plate (10⁴ cell/well) and incubated for 24 hours adding the substances to be assayed. Absorbance has been measured at 570 nm with an ELISA reader.

Further western blot has been carried out on Bcl2. The proteins have been extracted using buffer containing Tris-HCl 50 mM, pH 7,4, EDTA 2 mM, leuptin 100 µg/ml and 100 NaCl mM.

50 ug of proteins have been loaded and separated by SDS-PAGE. Monoclonal antibodies against Bcl-2, Bax and actin have been diluted 1:500, the antigen-antibody complex has been detected using ECL system and the result analyzed using image densitometry (Bio-Rad GS-700).

### Results and discussions

In the range of tested concentrations (0.01-0.5 µM) lunasin containing biomass induces an increase of in vitro DPCs proliferation according to dose dependent way (p<0.05) (Figura 1).

The effect of lunasin containing biomass, differently than biomass without lunasin, induces an increase of Bcl-2 protein expression and a decrease of Bax protein expression (Figura 2).

These data suggest that lunasin containing biomass stimulates the hair growth through proliferative and anti-apoptotic effect thereof on DPCs, could, therefore extend the anagen phase.

## Claims

1. Process for the preparation of a fermented soy based mixture, comprising isoflavones-aglicones, equol and lunasin by soy fermentation using a mixture of the following four lactic acid bacteria: *Lactobacillus plantarum* DSM 23755, *Lactobacillus plantarum* DSM 23756, *Lactobacillus fermentum* DSM 23757 and *Lactobacillus rhamnosus* DSM 23758.

2. Process according to claim 1, comprising or consisting of the following steps:
a) culture propagating said four *Lactobacillus plantarum* DSM 23755, *Lactobacillus plantarum* DSM 23756, *Lactobacillus fermentum* DSM 23757 and *Lactobacillus rhamnosus* DSM 23758 lactic acid bacteria;
b) inoculating soy based substrates with an aqueous suspension of said lactic acid bacteria;
c) incubating at 30 - 37°C, preferably 30°C, for 48 - 96 h, preferably 96 h.

3. Process according to claim 2, wherein the substrates are inoculated with a aqueous suspension of the lactic acid bacteria in an amount from 1 to 4% of total substrate volume, said aqueous suspension having a cell density of about log 9,0 cfu/ml for every strain.

4. Process according to claim 2, wherein soy based substrates are selected from the group consisting of soy flour, preferably organic farming soy flour, soy milk.

5. Process according to anyone of preceding claims further comprising step d) of centrifugation of broth-culture in order to remove the lactic acid bacteria cells.

6. Process according to claim 5, wherein the centrifugation of broth-culture is carried out at 10.000 x *g* for 15 min at 4°C.

7. Process according to anyone of preceding claims further comprising step e) of dehydration of the supernatant obtained in step d) or of culture obtained in step c) by drying or freeze-drying.

8. Fermented soy based mixture, comprising isoflavones-aglicones, equol and lunasin, obtainable by the process as defined in anyone of claims from 1 to 4, said process optionally further comprising a step e) of dehydration of the culture obtained in step c) by drying or freeze-drying, wherein said mixture comprises the following four lactic acid bacteria: *Lactobacillus plantarum* DSM 23755, *Lactobacillus plantarum* DSM 23756, *Lactobacillus fermentum* DSM 23757 and *Lactobacillus rhamnosus* DSM 23758.

9. Pharmaceutical or cosmetic composition comprising or consisting of the mixture as defined in claim 8 together with one or more pharmaceutical or cosmetically acceptable excipients and/or adjuvants.

10. Mixture according to claim 8, as such or in combination with one or more excipients and/or adjuvants, for use as food integrator.

11. Mixture according to claim 8 or composition according to claim 9 for use in the treatment of disorders or diseases of skin or intestinal wall.

12. Mixture according to claim 8 or composition according to claim 9 for cosmetic use.

13. Mixture for use according to claim 12 for the treatment of hair loss.

14. Mixture according to claim 8 or composition according to claim 9 for use in the treatment of alopecia or telogen defluvium.

15. Mixture of the following four lactic acid bacteria *Lactobacillus plantarum* DSM 23755, *Lactobacillus plantarum* DSM 23756, *Lactobacillus fermentum* DSM 23757 and *Lactobacillus rhamnosus* DSM 23758.

16. *Lactobacillus plantarum* DSM 23755 lactic acid bacterium

17. *Lactobacillus plantarum* DSM 23756 lactic acid bacterium.

18. *Lactobacillus fermentum* DSM 23757 lactic acid bacterium

19. *Lactobacillus rhamnosus* DSM 23758 lactic acid bacterium.

## Patentansprüche

1. Verfahren zur Herstellung eines fermentierten Soja basierten Gemisches, umfassend Isoflavone-Aglykone, Equol und Lunasin, durch Soja-Fermentation unter Verwendung eines Gemisches der nachstehenden vier Milchsäurebakterien: *Lactobacillus plantarum* DSM 23755, *Lactobacillus plantarum* DSM 23756, *Lactobacillus fermentum* DSM 23757 und *Lactobacillus rhamnosus* DSM 23758.

2. Verfahren nach Anspruch 1, umfassend oder bestehend aus den nachstehenden Schritten:
a) Kultur-Vermehrung der vier *Lactobacillus plantarum* DSM 23755-, *Lactobacillus plantarum* DSM 23756-, *Lactobacillus fermentum* DSM 23757- und *Lactobacillus rhamnosus* DSM 23758-Milchsäurebaktierien;
b) Inokulieren von Soja basierten Substraten mit einer wässrigen Suspension der Milchsäurebakterien;
c) Inkubieren bei 30 - 37°C, vorzugsweise 30°C, für 48 - 96 h, vorzugsweise 96 h.

3. Verfahren nach Anspruch 2, wobei die Substrate mit einer wässrigen Suspension der Milchsäurebakterien in einer Menge von 1 bis 4% vom Gesamtsubstratvolumen inokuliert werden, wobei die wässrige Suspension eine Zelldichte von etwa log 9,0 cfu/ml für jeden Stamm aufweist.

4. Verfahren nach Anspruch 2, wobei Soja basierte Substrate ausgewählt sind aus der Gruppe, bestehend aus Soja-Mehl, vorzugsweise organischem agrarwirtschaftlichem Soja-Mehl, Soja-Milch.

5. Verfahren nach einem der vorstehenden Ansprüche, weiter umfassend Schritt d) der Zentrifugierung von Kulturbrühe, um die Milchsäurebakterien-Zellen zu entfernen.

6. Verfahren nach Anspruch 5, wobei die Zentrifugierung von Kulturbrühe bei 10 000 x g für 15 min bei 4°C ausgeführt wird.

7. Verfahren nach einem der vorstehenden Ansprüche, weiter umfassend Schritt e) der Entwässerung des in Schritt d) erhaltenen Überstands oder von in Schritt c) durch Trocknen oder Gefrier-Trocknen erhaltener Kultur.

8. Fermentiertes auf Soja basiertes Gemisch, umfassend Isoflavone-Aglykone, Equol und Lunasin, erhältlich durch das wie in einem der Ansprüche 1 bis 4 definierte Verfahren, wobei das Verfahren gegebenenfalls weiter einen Schritt e) der Entwässerung der in Schritt c) durch Trocknen oder Gefrier-Trocknen erhaltenen Kultur umfasst, wobei das Gemisch die nachstehenden vier Milchsäurebakterien: *Lactobacillus plantarum* DSM 23755, *Lactobacillus plantarum* DSM 23756, *Lactobacillus fermentum* DSM 23757 und *Lactobacillus rhamnosus* DSM 23758 umfasst.

9. Pharmazeutische oder kosmetische Zusammensetzung, umfassend oder bestehend aus dem wie in Anspruch 8 definierten Gemisch zusammen mit einem oder mehreren pharmazeutisch oder kosmetisch verträglichen Exzipienten und/oder Adjuvantien.

10. Gemisch nach Anspruch 8, als solches oder in Kombination mit einem oder mehreren Exzipienten und/oder Adjuvantien, zur Verwendung als Nahrungsintegrator.

11. Gemisch nach Anspruch 8 oder Zusammensetzung nach Anspruch 9 zur Verwendung bei der Behandlung von Störungen oder Krankheiten der Haut oder der Darmwand.

12. Gemisch nach Anspruch 8 oder Zusammensetzung nach Anspruch 9 zur kosmetischen Verwendung.

13. Gemisch zur Verwendung nach Anspruch 12 für die Behandlung von Haarverlust.

14. Gemisch nach Anspruch 8 oder Zusammensetzung nach Anspruch 9 zur Verwendung bei der Behandlung von Alopezie oder Telogen defluvium.

15. Gemisch der nachstehenden vier Milchsäurebakterien *Lactobacillus plantarum* DSM 23755, *Lactobacillus plantarum* DSM 23756, *Lactobacillus fermentum* DSM 23757 und *Lactobacillus rhamnosus* DSM 23758.

16. *Lactobacillus plantarum* DSM 23755 Milchsäurebakterium.

17. *Lactobacillus plantarum* DSM 23756 Milchsäurebakterium.

18. *Lactobacillus fermentum* DSM 23757 Milchsäurebakterium.

19. *Lactobacillus rhamnosus* DSM 23758 Milchsäurebakterium.

## Revendications

1. Procédé pour la préparation d'un mélange à base de soja fermenté, comprenant des isoflavones-aglycones, de l'équol et de la lunasine par fermentation de soja en utilisant un mélange des quatre bactéries d'acide lactique suivantes : *Lactobacillus plantarum* DSM 23755, *Lactobacillus plantarum* DSM 23756, *Lactobacillus fermentum* DSM 23757 et *Lactobacillus rhamnosus* DSM 23758.

2. Procédé selon la revendication 1, comprenant ou constitué des étapes suivantes :
a) propagation de la culture desdites quatre bactéries d'acide lactique *Lactobacillus plantarum* DSM 23755, *Lactobacillus plantarum* DSM 23756, *Lactobacillus fermentum* DSM 23757 et *Lactobacillus rhamnosus* DSM 23758 ;
b) inoculation de substrats à base de soja avec une suspension aqueuse desdites bactéries d'acide lactique ;
c) incubation à 30 à 37 °C, de préférence à 30 °C, pendant 48 à 96 h, de préférence à 96 h.

3. Procédé selon la revendication 2, dans lequel les substrats sont inoculés avec une suspension aqueuse des bactéries d'acide lactique en quantité de 1 à 4 % du volume total du substrat, ladite suspension aqueuse ayant une densité de cellules d'environ log 9,0 cfu/ml pour chaque souche.

4. Procédé selon la revendication 2, dans lequel les substrats à base de soja sont choisis dans le groupe constitué de la farine de soja, de préférence de la farine de soja de culture organique, du lait de soja.

5. Procédé selon l'une quelconque des revendications précédentes, comprenant en outre l'étape d) de centrifugation d'un bouillon de culture afin d'éliminer les cellules de bactéries d'acide lactique.

6. Procédé selon la revendication 5, dans lequel la centrifugation du bouillon de culture est effectuée à 10 000 x g pendant 15 min à 4 °C.

7. Procédé selon l'une quelconque des revendications précédentes, comprenant en outre l'étape e) de déshydratation du surnageant obtenu à l'étape d) ou de la culture obtenue à l'étape c) par séchage ou lyophilisation.

8. Mélange à base de soja fermenté, comprenant des isoflavones-aglycones, de l'équol et de la lunasine, que l'on peut obtenir par le procédé selon l'une quelconque des revendications 1 à 4, ledit procédé comprenant en outre éventuellement une étape e) de déshydratation de la culture obtenue à l'étape c) par séchage ou lyophilisation, dans lequel ledit mélange comprend les quatre bactéries d'acide lactique suivantes : *Lactobacillus plantarum* DSM 23755, *Lactobacillus plantarum* DSM 23756, *Lactobacillus fermentum* DSM 23757 et *Lactobacillus rhamnosus* DSM 23758.

9. Composition pharmaceutique ou cosmétique comprenant ou constituée du mélange selon la revendication 8 conjointement avec un ou plusieurs excipients et/ou adjuvants acceptables au plan pharmaceutique ou cosmétique.

10. Mélange selon la revendication 8, tel quel ou en combinaison avec un ou plusieurs excipients et/ou adjuvants pour utilisation comme intégrateur alimentaire.

11. Mélange selon la revendication 8 ou composition selon la revendication 9, pour utilisation dans le traitement de troubles ou de maladies de la peau ou de la paroi intestinale.

12. Mélange selon la revendication 8 ou composition selon la revendication 9 pour utilisation cosmétique.

13. Mélange pour utilisation selon la revendication 12 pour le traitement de la perte de cheveux.

14. Mélange selon la revendication 8 ou composition selon la revendication 9 pour utilisation dans le traitement de l'alopécie ou de l'effluvium télogène.

15. Mélange des quatre bactéries d'acide lactique suivantes : *Lactobacillus plantarum* DSM 23755, *Lactobacillus plantarum* DSM 23756, *Lactobacillus fermentum* DSM 23757 et *Lactobacillus rhamnosus* DSM 23758.

16. Bactérie d'acide lactique *Lactobacillus plantarum* DSM 23755.

17. Bactérie d'acide lactique *Lactobacillus plantarum* DSM 23756.

18. Bactérie d'acide lactique *Lactobacillus fermentum* DSM 23757.

19. Bactérie d'acide lactique *Lactobacillus rhamnosus* DSM 23758.


### SEQUENCE LISTING

<110> Giuliani S.p.A.
<120> Fermented soya based mixture comprising isoflavones-aglycones, equol and lunasin, process for the preparation thereof and related uses in food, medical and cosmetic fields
<130> PCT28360
<150> RM2010A000378
   <151> 2010-07-12
<160> 2
<170> PatentIn version 3.5
<210> 1
   <211> 10
   <212> DNA
   <213> primer for the random amplification of chromosomal and pasmidic bacteria
   DNA
<400> 1
   agcagcgtgg 10
<210> 2
   <211> 15
   <212> DNA
   <213> artificial
<220>
   <223> primer for the random amplification of chromosomal and pasmidic bacteria DNA
<400> 2
   gagggtggcg gttct 15